**Europäisches Patentamt**

**European Patent Office**

**Office européen des brevets**

(19)

(11) Numéro de publication: **0 175 595**
**B1**

(12)

# FASCICULE DE BREVET EUROPEEN

(45) Date de publication du fascicule du brevet:
**19.11.87**

(51) Int. Cl.⁴: **A 61 M 1/00, A 61 M 27/00**

(21) Numéro de dépôt: **85400847.1**

(22) Date de dépôt: **30.04.85**

(54) **Sonde viscérale auto-statique à revêtement helicoidal.**

(30) Priorité: **24.07.84 FR 8411709**

(43) Date de publication de la demande:
**26.03.86 Bulletin 86/13**

(45) Mention de la délivrance du brevet:
**19.11.87 Bulletin 87/47**

(84) Etats contractants désignés:
**AT BE CH DE FR GB IT LI LU NL SE**

(56) Documents cités:
**CH - A - 84 707**
**FR - A - 462 423**
**FR - A - 564 832**
**FR - A - 585 573**

(73) Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Levy, Etienne, 106, avenue de Villiers, F-75017 Paris (FR)**
Inventeur: **Honiger, Jiri, 14/16, rue Louis Michel, F-94800 Villejuif (FR)**
Inventeur: **Parc, Rolland, 10, avenue Léon Bourgain, F-92400 Courbevoie (FR)**
Inventeur: **Ollivier, Jean-Marc, 22, rue André Bollier, F-94100 Saint-Maur (FR)**

(74) Mandataire: **Ores, Irène et al, CABINET ORES 6, Avenue de Messine, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à une sonde viscérale pour l'évacuation d'un liquide biologique hors du corps, ou pour l'instillation d'un liquide thérapeutique dans le corps, ou encore pour l'évacuation d'un liquide biologique et l'instillation d'un liquide thérapeutique effectuées simultanément ou alternativement.

On entend par sonde viscérale, dans le sens de la présente invention, une sonde, une canule ou un drain.

On connaît de nombreux dispositifs de sonde assurant l'évacuation ou l'instillation de liquides dans les circonstances les plus différentes de la thérapeutique médico-chirurgicale.

Le Brevet français N° 2 240 026 décrit, par exemple, un drain à usage médical, réalisé en matériau souple ou semi-rigide, opaque ou non, notamment en caoutchoucs naturels ou synthétiques, en chlorure de polyvinyle ou en élastomères, tels que les élastomères de silicones, dont le débit du liquide biologique drainé, ou le débit du liquide d'irrigation de la région drainée, est augmenté en ménageant sur la surface externe du drain, de façon plus précise sur une partie de sa longueur jusqu'à l'extrémité distale, une ou plusieurs rainures, de préférence à bords arrondis et à section sensiblement constante notamment, qui communiquent avec le canal central par une série d'orifices latéraux, en particulier sensiblement circulaires, disposés à l'intérieur desdites rainures.

En particulier, ces rainures peuvent être hélicoïdales et l'extrémité du drain peut être fermée.

Un autre Brevet français N° 2 450 614 fait connaître un drain d'aspiration d'un liquide biologique à usage médical, du type consistant en un tuyau flexible, qui permet d'éliminer aisément les colmatages des perforations distales et/ou de la lumière du canal central du drain, lesquels colmatages peuvent être provoqués par la coagulation du liquide biologique pendant les intervalles où il n'y a presque pas d'extraction de ce dernier.

A cet effet, ce deuxième Brevet propose, en particulier, la réalisation, au niveau de l'extrémité distale d'un tel drain, d'une perforation présentant la configuration d'une gorge hélicoïdale qui, lors de l'introduction du drain à l'intérieur du corps, effectuée par un mouvement de rotation, subit un rétrécissement capable de briser un éventuel colmatage se produisant lors de l'implantation du drain (au contraire, l'extraction de ce drain, obtenue en inversant le sens de rotation, détermine un élargissement de ladite gorge hélicoïdale) ; en outre, la présence d'une telle gorge hélicoïdale donne à la lumière de l'extrémité distale une configuration en «trou de serrure» à laquelle correspondent des forces d'adhérence du liquide biologique moins fortes que pour une section circulaire, ce qui s'oppose au colmatage de cette lumière.

Toutefois, les dispositifs de sonde de l'Art antérieur qui précèdent présentent tous, mise à part leur portion distale, une surface extérieure lisse, ou exceptionnellement granitée, et sont fixés selon une méthode invasive, éphémère et fragile, à l'aide de fils amarrés par des points trans-fixiants tégumentaires souvent douloureux et rapidement exposés à deux risques majeurs :
– l'absence d'étanchéité à la jonction avec un conduit naturel ou avec les téguments,
– et, surtout, l'absence de fixation efficace assurant le maintien du dispositif dans la position initiale prévue par l'opérateur. En effet, le dispositif de sonde est exposé à l'expulsion, soit par les mouvements intempestifs du malade ou de l'entourage, soit par le péristaltisme naturel s'il s'agit d'une intubation intra-luminale d'un viscère creux.

Or, l'arrachage et l'expulsion d'un dispositif de sonde constituent un risque toujours grave pour le patient :
– entraînement de dégâts viscéraux parfois importants ;
– suppression, de façon souvent définitive, d'une voie thérapeutique salvatrice, par exemple dans le cas d'une jéjunostomie difficile d'alimentation chez un sujet poly-opéré, et
– remise en place obligatoire par voie généralement sanglante.

Il est vrai qu'auparavant les brevets français N° 462 423 et 564 832 divulgaient la possibilité de réaliser, respectivement :
– un drain armé hélicoïdal, comportant un tube en caoutchouc pourvu à sa surface externe d'une dépression hélicoïdale séparant les spires d'un relief hélicoïdal (l'armature étant constituée par un fil métallique enroulé en hélice et noyé dans les spires) et d'une pluralité d'orifices latéraux de communication avec l'extérieur ménagés entre les spires, et
– un drain en caoutchouc pourvu sur sa paroi externe de filets pleins, également en caoutchouc, qui sont disposés en hélice et dont le nombre, le diamètre et le pas peuvent être variables,
toutefois les possibilités de traitement de ces derniers dispositifs étaient limitées au seul drainage.

La présente invention a, en conséquence, pour but de pourvoir à un dispositif de sonde, de canulation ou de drainage de liquide biologique qui répond mieux aux nécessités de la pratique que les dispositifs visant au même but antérieurement connus, notamment en ce que :
– il est spontanément auto-statique,
– il est inextirpable accidentellement, à savoir résistant à l'expulsion par les mouvements péristaltiques ou par les mouvements intempestifs du malade et de l'entourage ;
– sa fixation est non traumatisante, stable et durable ;
– il permet également la fixation trans-pariétale d'une sonde intra-viscérale, par exemple dans le cas du thorax ou de l'abdomen, laquelle fixation trans-pariétale est actuellement obtenue par collage ou par points trans-fixiants sanglants d'efficacité transitoire ;
– la jonction avec le conduit naturel ou la zone intubée est étanche, et
– le coût du dispositif de sonde selon l'invention, destiné à usage clinique, est faible.

La présente invention a pour objet un dispositif de sonde, de canulation ou de drainage de liquide biologique, du type réalisé en une matière souple, notamment en élastomère de silicone, et présentant:

—une ou plusieurs dépressions ou rainures hélicoïdales qui, étant ménagées à sa surface externe et décalées l'une par rapport à l'autre dans le sens de la longueur, séparent des spires appartenant à un ou plusieurs reliefs hélicoïdaux de pas constant ou variable, et

—éventuellement, des orifices latéraux de communication avec l'extérieur du dispositif, lesquels orifices sont ménagés dans ladite ou lesdites dépressions hélicoïdales, et en particulier à l'extrémité distale,

lequel dispositif est caractérisé en ce que ledit ou lesdits reliefs hélicoïdaux, et donc lesdites spires, sont creux, de façon telle que les spires intercommuniquant entre elles, à savoir appartenant à un même relief, définissent un ou plusieurs canaux périphériques indépendants qui communiquent chacun, à l'extrémité proximale, avec la lumière d'une tubulure latérale raccordée à une prise d'air et/ou d'une source de liquide d'instillation ou d'irrigation, tandis qu'à l'extrémité distale au moins un desdits canaux périphériques est en communication avec l'intérieur ou l'extérieur du dispositif par l'intermédiaire d'un orifice latéral.

Le dispositif de sonde, de canulation ou de drainage est apte à être introduit dans la zone concernée par une rotation à la façon d'un vissage avec introduction non traumatisante, étanche et autostatique, par contrainte périphérique desdites spires contre le conduit naturel ou la paroi musculoaponévrotique concernés.

Selon un mode de réalisation avantageux du dispositif conforme à l'invention, lorsque celui-ci est du type présentant un ou plusieurs reliefs hélicoïdaux rapportés, à savoir constitués par un ou plusieurs filets indépendants souples qui sont enroulés en hélice autour d'un tube central à extrémité distale ouverte, de forme cylindrique, cylindro-conique, tronconique, ovale ou autre et délimitant un canal central de drainage, et qui sont solidarisés de ce tube central par tout moyen approprié et en particulier par collage, ledit dispositif est caractérisé en ce que ledit ou lesdits filets indépendants souples sont constitués par des tubulures hélicoïdales périphériques, dont la lumière définit ledit ou lesdits canaux périphériques indépendants.

Selon un mode de réalisation préféré du dispositif conforme à l'invention, lorsque celui-ci est du type présentant à sa surface externe une dépression ou rainure hélicoïdale séparant les spires d'un relief hélicoïdal externe intégré dans la paroi d'un tube périphérique souple, de forme cylindrique, cylindro-conique, tronconique, ovale ou autre, ledit dispositif est caractérisé en ce que ce tube périphérique présente également à la surface interne de sa paroi, d'épaisseur sensiblement constante, une dépression ou rainure hélicoïdale séparant les spires d'un relief hélicoïdal interne, cette dépression et ce relief hélicoïdaux internes correspondant, respectivement, auxdits relief et dépression hélicoïdaux externes, et en ce que le tube périphérique ayant une telle configuration hélicoïdale également sur sa surface intérieure constitue un revêtement hélicoïdal à hélice simple pour un tube central à extrémité distale ouverte, qui délimite un canal central et s'adapte à la forme du tube périphérique et dont le diamètre extérieur est sensiblement égal au diamètre intérieur que le tube périphérique présente au niveau dudit relief interne, de manière que ledit canal périphérique est compris entre la surface extérieure du tube central et la surface délimitant ladite dépression hélicoïdale interne, la solidarisation entre le tube central et le tube périphérique étant obtenue par tout moyen approprié, notamment par collage, en correspondance de ladite dépression hélicoïdale externe, à savoir en correspondance dudit relief hélicoïdal interne.

Selon un autre mode de réalisation préféré du dispositif conforme à l'invention, celui-ci comporte un revêtement hélicoïdal qui est constitué par un tube souple périphérique enveloppant un tube central du type susdit et présentant à sa surface externe et à sa surface interne une configuration à pas multispire, à savoir une pluralité de dépressions hélicoïdales externes et internes indépendantes décalées l'une par rapport à l'autre dans le sens de la longueur, qui séparent les spires d'une égale pluralité de reliefs hélicoïdaux indépendants externe et interne, dont la surface interne délimite lesdits canaux périphériques indépendants en coopération avec la surface extérieure dudit tube central, cette double pluralité de dépressions hélicoïdales et de reliefs hélicoïdaux, internes et externes, étant obtenue en une seule pièce.

Selon un autre mode de réalisation avantageux du dispositif conforme à l'invention, les portions terminales des extrémités distale et proximale du tube périphérique, qui enveloppent le tube central, sont conformées de manière à ne pas comporter de spires et à s'appliquer par la totalité de leur surface interne, sur la surface externe des portions terminales des extrémités distale et proximale correspondantes du tube central, lesdites portions terminales respectives étant solidarisées entre elles par tous moyens appropriés, et notamment par collage.

Selon un autre mode de réalisation avantageux du dispositif conforme à l'invention, dans le cas où le relief hélicoïdal est constitué par ladite ou lesdites tubulures hélicoïdales périphériques, l'extrémité distale de cette ou de ces tubulures périphériques est fermée, par collage notamment, et s'applique sur l'extrémité distale correspondante dudit tube central qui se prolonge dans une portion terminale ne comportant pas de spires.

Selon un autre mode de réalisation avantageux du dispositif conforme à l'invention, également la portion terminale du tube central dépourvue de spires, qui se trouve du côté distal, est pourvue d'orifices latéraux.

Selon un autre mode de réalisation avantageux du dispositif conforme à l'invention, ledit orifice latéral qui met en communication, au niveau de l'extrémité distale ledit ou lesdits canaux périphériques avec ledit canal central est muni d'un clapet anti-reflux.

Selon un autre mode de réalisation avantageux du dispositif conforme à l'invention, les spires de ladite ou desdites tubulures hélicoïdales périphériques et dudit revêtement hélicoïdal présentant une double paroi, de façon à délimiter deux canaux périphériques hélicoïdaux juxtaposés, à savoir un premier canal hélicoïdal compris entre la double paroi et le tube central, et un deuxième canal hélicoïdal compris dans ladite double paroi.

Selon une disposition avantageuse de ce mode de réalisation, le diamètre dudit deuxième canal périphérique hélicoïdal, à savoir le diamètre des spires, est rendu variable à l'aide d'un système de réglage approprié relié, à l'aide d'un tube latéral, à ladite double paroi, le deuxième canal périphérique ne communiquant pas avec le premier canal périphérique ni avec ledit canal central.

Selon une modalité avantageuse de cette disposition, le système de réglage du diamètre des spires est constitué par un système pneumatique communiquant avec ledit deuxième canal hélicoïdal compris dans ladite double paroi.

Selon une autre modalité avantageuse de cette disposition, le système de réglage du diamètre des spires est constitué par un dispositif de coulage, dans ledit canal hélicoïdal de la double paroi, d'une mousse en matière plastique.

Selon un autre mode de réalisation avantageux du dispositif conforme à l'invention, la paroi du tube central présente, du côté proximal, un épaississement permettant son raccordement à un collecteur du liquide biologique en dépression, par l'intermédiaire d'un connecteur.

Selon encore un autre mode de réalisation avantageux du dispositif conforme à l'invention, l'extrémité distale du tube central est biseautée, notamment selon un profil tronconique sur lequel s'adapte ledit revêtement hélicoïdal.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels:
—la figure 1 est une vue de côté, avec arrachements partiels, d'un premier mode de réalisation préféré du dispositif de sonde conforme à la présente invention;
—la figure 2 est une vue de côté, avec arrachements partiels, d'un deuxième mode de réalisation avantageux du dispositif de sonde selon l'invention;
—la figure 3 représente le dispositif de sonde, conforme à la figure 1, appliqué à une jéjunostomie; et
—la figure 4 représente une vue de côté, avec arrachement partiel, d'un troisième mode de réalisation préféré du dispositif de sonde selon l'invention.

Il doit être bien entendu, toutefois, que ces dessins et les parties descriptives correspondantes, sont donnés uniquement à titre d'illustration de l'objet de l'invention, dont ils ne constituent en aucune manière une limitation.

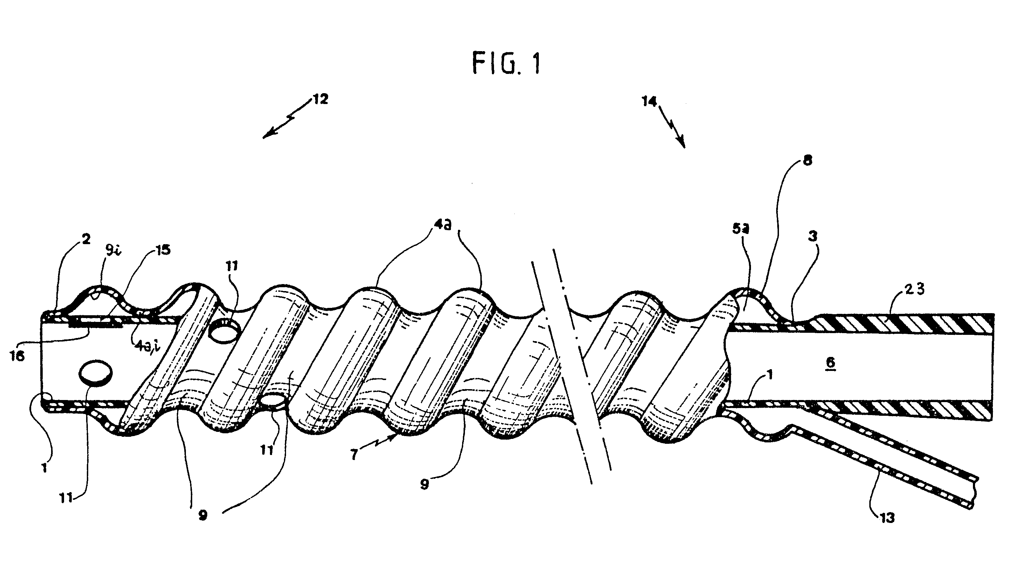

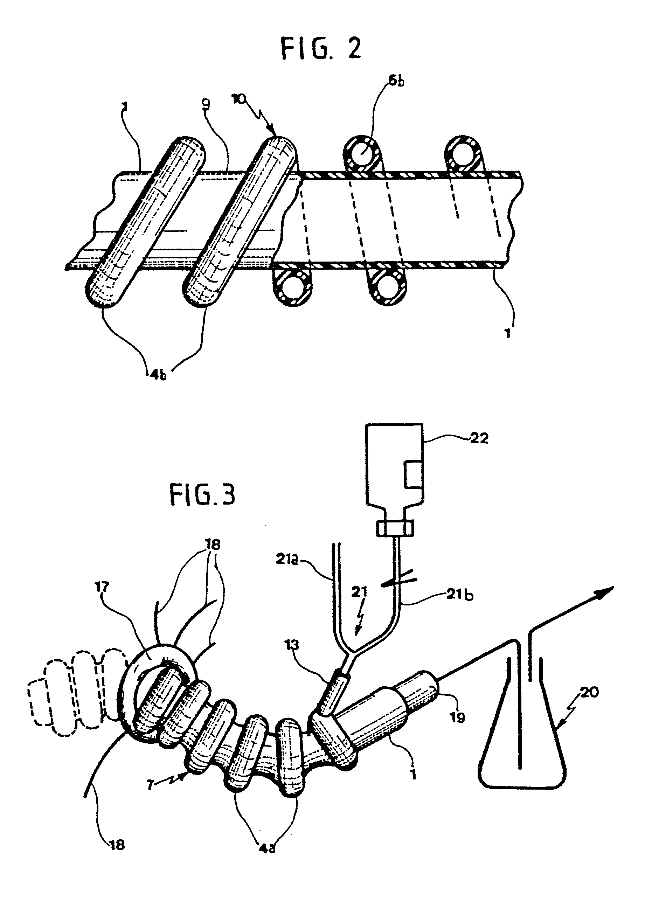

La sonde viscérale conforme à la présente invention et illustrée aux figures 1 et 2 présente, sur toute la longueur utile de son tube central 1, sauf sur les deux parties terminales 2 et 3 situées du côté distal et proximal, respectivement, de la sonde, un relief hélicoïdal comportant des spires creuses 4a, 4b écartées l'une par rapport à l'autre du pas de l'hélice définie par le relief hélicoïdal.

Ce relief hélicoïdal permet l'introduction par un simple mouvement non traumatisant, à la façon d'un vissage, de la partie distale de la sonde dans un conduit naturel, comme dans le cas de stomies intestinales, biliaires ou urinaires, artificielles ou pathologiques, ou l'introduction dans une paroi musculo-aponévrotique: cette introduction assure, d'une part, une fixation auto-statique qui supprime les risques d'arrachage ou de retrait accidentels dus aux mouvements intempestifs du malade ou de son entourage, ou involontaires dus aux péristaltisme naturel s'il s'agit d'une intubation intra-luminale d'un viscère creux, ainsi que, d'autre part, une jonction étanche et sans intolérance entre la sonde et la zone intubée, grâce à la contrainte périphérique non traumatisante desdites spires 4a, 4b contre le conduit naturel ou la paroi musculo-aponévrotique concernés.

Les spires hélicoïdales creuses 4a, 4b délimitent un canal périphérique hélicoïdal 5a, 5b qui peut être destiné à coopérer avec le canal 6 dudit tube central 1, comme il sera mieux décrit par la suite.

En outre, les spires du relief hélicoïdal peuvent présenter une double paroi (non représentée), de façon à délimiter deux canaux périphériques hélicoïdaux, à savoir un premier canal hélicoïdal, compris entre la double paroi et le tube central, et un deuxième canal hélicoïdal compris dans ladite double paroi.

A titre d'exemple non limitatif, le pas entre les spires 4a, 4b est compris entre 5 et 20 mm, tandis que l'épaisseur transversale de ces spires est comprise entre 2 et 10 mm, selon les applications.

Selon un premier mode de réalisation préféré représenté à la figure 1, le relief hélicoïdal est constitué par un tube souple périphérique 7, dont la paroi 8 présente une configuration hélicoïdale non seulement à la surface externe mais également à sa surface interne (au relief hélicoïdal externe correspond, à l'intérieur, la dépression hélicoïdale interne 9i, tandis qu'à la dépression hélicoïdale externe 9 correspond, à l'intérieur, un relief hélicoïdal interne définissant les spires internes 4a,i). Le revêtement 7 enveloppe le tube central 1.

La solidarisation entre ce dernier et le tube périphérique 7 se fait, par collage notamment, en correspondance des portions 9 du tube 7 qui sont comprises entre ses spires consécutives 4a et qui adhèrent à la surface extérieure du tube central 1, en sorte qu'entre cette dernière et la surface intérieure du tube périphérique 7 est délimité un canal périphérique hélicoïdal 5a.

Les portions terminales 2 et 3 des extrémités distale 12 et proximale 14, respectivement, du tube périphérique 7, qui enveloppent le tube central 1, sont conformées de manière à ne pas comporter de spires et à s'appliquer par la totalité de leur surface interne sur la surface externe des portions terminales des extrémités distale et proximale correspondantes dudit tube central 1, les portions terminales respectives étant solidarisées entre elles par tous moyens appropriés, et notamment par collage.

Conformément à un deuxième mode de réalisation avantageux de la sonde viscérale selon l'invention, représentée à la figure 2, ledit relief hélicoïdal est constitué par un tube souple 10 qui est enroulé en hélice, selon un pas déterminé, autour du tube central 1 et qui est solidarisé avec ce dernier, par exemple encore par collage: dans ce cas un canal périphérique hélicoïdal 5b est donc défini par la lumière du tube 10 ainsi enroulé.

L'extrémité distale du tube périphérique 10 enroulé autour du tube central 1 est fermée, par collage notamment, et s'applique sur l'extrémité distale dudit tube central 1, qui se prolonge dans une portion terminale ne comportant pas de spires.

Dans chaque mode de réalisation de la sonde viscérale, le tube central 1, ainsi que les tubes périphériques 7, 10, sont réalisés en matière plastique souple, notamment en élastomères de silicones, telle que la sonde présente une souplesse lui permettant d'adopter des courbures importantes sans toutefois se plier et s'écraser (cf. la figure 3).

En outre, dans chacun de ces modes de réalisations, les orifices latéraux 11 (cf. la figure 1), dont est généralement pouvue l'extrémité distale 12 de la sonde, sont ménagés entre les spires (cf. la figure 1) et mettent en communication le canal central 6 avec l'extérieur de la sonde: évidemment, dans le cas de la figure 1, les orifices latéraux 11 traversent également la paroi 8 du tube périphérique 7.

Des orifices latéraux (non représentés) peuvent aussi être ménagés sur ladite partie terminale 2, dépourvue de revêtement hélicoïdal, qui se trouve du côté distal.

En outre, l'extrémité distale 12 du tube central 1 peut être biseautée, notamment selon un profil tronconique (non représenté), sur lequel s'adaptent le revêtement hélicoïdal 7 ou le tube 10.

Un tube latéral 13 (cf. les figures 1 et 3) est raccordé au tube périphérique 7 en correspondance de l'extrémité proximale 14, faisant ainsi communiquer le canal hélicoïdal 5a avec l'atmosphère, ce qui permet:
— de disposer d'une prise d'air à la pression atmosphérique qui favorise l'évacuation du liquide biologique, située dans le conduit naturel ou dans la paroi musculo-aponévrotique concernés, par siphonnage ou par aspiration sous dépression modérée, et/ou
— de réaliser une irrigation continue ou séquentielle avec un liquide diluant ou thérapeutique, si l'évacuation de liquide biologique hétérogène ou consistant l'exige: à cet effet le canal hélicoïdal est branché sur une source de liquide d'irrigation (ou instillation).

Le canal périphérique hélicoïdal 5a, 5b communique, en correspondance de l'extrémité distale 12 du tube central 1, avec le canal central 6 par un orifice latéral 15 qui est muni d'un clapet anti-reflux 16, ce qui empêche le liquide biologique de la zone concernée par l'intubation de la sonde:
— de pénétrer dans le canal hélicoïdal 5a, 5b;
— d'obturer ladite prise d'air;
— d'augmenter la perte de charge dans cette prise d'air, et
— de diminuer ainsi la sécurité attendue.

Dans le cas où le tube périphérique 7 ou 10 présente une double paroi, le canal hélicoïdal compris dans cette dernière est relié à un système (non représenté) assurant le réglage du diamètre des spires 4a ou 4b, en fonction des conditions d'emploi.

Ce système de réglage peut être constitué par un système pneumatique communiquant avec le canal hélicoïdal compris dans ladite double paroi, ou par un dispositif de coulage d'une mousse en matière plastique dans ce même canal hélicoïdal.

La figure 3 montre l'intubation d'une stomie 17 du jéjunum, fixée à la peau par des fils 18 à l'aide de la sonde viscérale auto-statique à relief hélicoïdal, en particulier conforme à la réalisation selon la figure 1.

L'extrémité proximale 12 du tube central 1, qui présente à cet effet une zone plus épaisse 23, est raccordée par l'intermédiaire d'un connecteur 19, à un collecteur 20 du liquide biologique en dépression; en outre, cette extrémité proximale est raccordée, par l'intermédiaire du tube latéral 13, à une tubulure 21 en forme de Y dont un segment 21a est en communication avec l'air, pour constituer une prise d'air à la pression atmosphérique, tandis que l'autre segment 21b est relié à un flacon 22 d'instillation par gravité de la région du jéjunum intéressée par l'intubation.

Le relief hélicoïdal de la sonde viscérale offre, dans le cas particulier d'une canulation jéjuno-iléale, l'avantage exceptionnel, jamais réalisé jusqu'ici, d'une neutralisation mécanique des forces mises en jeu par le péristaltisme intestinal perpendiculairement à l'axe de la sonde et dont les composantes en amont et en aval du profil hélicoïdal s'annulent respectivement.

La sonde se révèle également efficace en réanimation chirurgicale digestive en cas de stomie jéjunale haute hyperproductive.

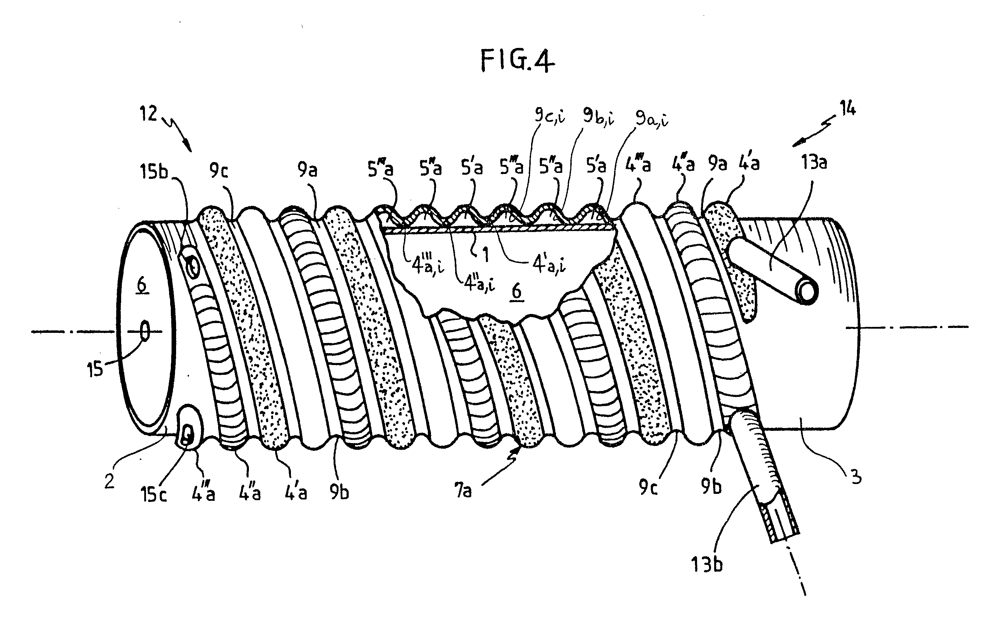

Le dispositif de sonde représenté à la figure 4 correspond à un autre mode de réalisation préféré et comporte un tube central 1 qui est enveloppé par un revêtement réalisé en une seule pièce et constitué par un tube périphérique 7a à pas multispire, à savoir qui présente une pluralité de dépressions hélicoïdales indépendantes 9a, 9b, 9c et décalées l'une par rapport à l'autre dans le sens de la longueur.

Ces dépressions séparent une pluralité de reliefs hélicoïdaux creux indépendants (pour des raisons de clarté du dessin, on a distingué les trois reliefs hélicoïdaux creux séparés par lesdites dépressions hélicoïdales indépendantes 9a, 9b, 9c par des signes ou traits différents) définissant une pluralité de spires creuses également indépendantes 4'a, 4''a, 4'''a qui délimitent une pluralité de canaux périphériques encore également indépendants 5'a, 5''a, 5'''a communiquant, à l'extrémité proximale 14, avec la lumière de trois tubulures latérales parmi lesquelles sont visibles sur la figure 4 seulement les tubulures 13a et 13b correspondant aux canaux 5'a et 5''a.

(On peut remarquer qu'aux dépressions hélicoïdales indépendantes externes 9a, 9b, 9c correspondent, à l'intérieur, des reliefs hélicoïdaux également indépendants définissant des spires internes 4'a,i, et 4'''a,i, tandis qu'aux spires indépendantes externes 4'a, 4''a et 4'''a, correspondent, à l'intérieur, des dépressions hélicoïdales également indépendantes 9a,i, 9b,i et 9c,i.)

Les tubulures 13a et 13b susdites sont susceptibles d'être raccordées, l'une d'entre elles à une source de vide, tandis qu'une autre à une prise d'air et la troisième à une source d'instillation ou d'irrigation.

Lorsqu'il est nécessaire d'assurer la circulation simultanée et indépendante de deux ou plusieurs fluides physiologiques (tandis qu'un canal périphérique est destiné à assurer la prise d'air nécessaire au drainage s'effectuant par le canal central 6), il suffit de destiner à cette fonction deux ou plusieurs canaux périphériques: en fait, bien que le dessin annexé illustre un dispositif de sonde comportant trois seuls canaux périphériques, il va de soi qu'on peut augmenter leur nombre selon les nécessités de la pratique clinique, notamment en réanimation polyvalente et spécialisée.

Lesdits canaux périphériques indépendants 5'a, 5''a, 5'''a peuvent déboucher, à la partie distale 12 dans le canal central 6, comme par exemple le canal 5'a par l'intermédiaire de l'orifice latéral 15 qui assure la prise d'air nécessaire, ou à l'extérieur de la sonde, comme par exemple les canaux 5''a, 5'''a par l'intermédiaire des orifices latéraux 15b et 15c qui assurent l'irrigation simultanée et indépendante de la zone concernée par le drainage à l'aide de deux fluides physiologiques appropriés.

Il est évident que l'avantage essentiel de la sonde de figure 4 consiste dans le fait de réaliser, à la surface d'un tube de drainage, une pluralité de reliefs indépendants creux à l'aide d'une pièce unique constituée par un revêtement hélicoïdal à pas multispire 7a, réalisé en une seule pièce, au lieu de réaliser ladite pluralité de tubulures indépendantes enroulées en hélice autour dudit tube de drainage et solidarisées, notamment par collage ou autre, à la surface externe de ce dernier.

Il va de soi encore que, bien que la description de la figure 4 se réfère à un tube central 6 et à un revêtement hélicoïdal à pas multispire 7a de forme cylindrique, elle s'applique également à toute autre forme, notamment cylindro-conique, tronconique, ovale, etc.

## Revendications

1. Dispositif de sonde, de canulation ou de drainage de liquide biologique, du type réalisé en une matière souple, notamment en élastomère de silicone, et présentant:
— une ou plusieurs dépressions ou rainures hélicoïdales (9 ou 9a, 9b, 9c) qui, étant ménagées à sa surface externe et décalées l'une par rapport à l'autre dans le sens de la longueur, séparent des spires (4a ou 4b ou 4'a, 4''a, 4'''a) appartenant à un ou plusieurs reliefs hélicoïdaux de pas constant ou variable, et
— éventuellement, des orifices latéraux (11) de communication avec l'extérieur du dispositif, lesquels orifices sont ménagés dans ladite ou lesdites dépressions hélicoïdales (9), et en particulier à l'extrémité distale (12),
lequel dispositif est caractérisé en ce que ledit ou lesdits reliefs hélicoïdaux, et donc lesdites spires (4a ou 4b ou 4'a, 4''a, 4'''a), sont creux, de façon telle que les spires intercommuniquant entre elles, à savoir appartenant à un même relief, définissent un ou plusieurs canaux périphériques (5a ou 5b ou 5'a, 5''a, 5'''a) indépendants qui communiquent chacun, à l'extrémité proximale (14), avec la lumière d'une tubulure latérale (13 ou 13a, 13b) raccordée à une prise d'air et/ou à une source de liquide d'instillation ou d'irrigation, tandis qu'à l'extrémité distale (12) au moins un desdits canaux périphériques est en communication avec l'intérieur ou l'extérieur du dispositif par l'intermédiaire d'un orifice latéral ( 15 ou 15b, 15c).

2. Dispositif selon la revendication 1, du type présentant un ou plusieurs reliefs hélicoïdaux rapportés, à savoir constitués par un ou plusieurs filets indépendants souples qui sont enroulés en hélice autour d'un tube central (1) à extrémité distale ouverte, de forme cylindrique, cylindro-conique, tronconique, ovale ou autre et délimitant un canal central (6) de drainage, et qui sont solidarisés de ce tube central (1) par tout moyen approprié et en particulier par collage, caractérisé en ce que ledit ou lesdits filets indépendants souples sont constitués par des tubulures hélicoïdales périphériques (10) dont la lumière définit ledit ou lesdits canaux périphériques indépendants.

3. Dispositif selon la revendication 1, du type présentant à sa surface externe une dépression hélicoïdale (9) séparant les spires (4a) d'un relief hélicoïdal externe intégré dans la paroi d'un tube périphérique souple, de forme cylindrique, cylindro-conique, tronconique, ovale ou autre, caractérisé en ce que ce tube périphérique (7) présente également à la surface interne de sa paroi, d'épaisseur sensiblement constante, une dépression ou rainure hélicoïdale (9i) séparant les spires (4a,i) d'un relief hélicoïdal interne, cette dépression et ce relief hélicoïdaux internes correspondant, respectivement, auxdits relief et dépression (9) hélicoïdaux externes, et en ce que le tube périphérique (7) ayant une telle configuration hélicoïdale également sur sa surface intérieure constitue en revêtement hélicoïdal à hélice simple pour un tube central (1) à extrémité distale ouverte, qui délimite un canal central (6) et s'adapte à la forme de tube périphérique (7) et dont le diamètre extérieur est sensiblement égal au diamètre intérieur que le tube périphérique présente au niveau dudit relief interne, de manière que ledit canal périphérique (5a) est compris entre la surface extérieure du tube central (1) et la surface délimitant ladite dépression hélicoïdale interne (9i), la solidarisation entre le tube central (1) et le tube périphérique (7) étant obtenue par tout moyen approprié, notamment par collage, en correspondance de ladite dépression hélicoïdale externe (9), à savoir en correspondance dudit relief hélicoïdal interne.

4. Dispositif selon la revendication 1, caractérisé en ce que celui-ci comporte un revêtement hélicoïdal qui est constitué par un tube souple périphérique (7a) enveloppant un tube central (1) du type susdit et présentant à sa surface externe et à sa surface interne une configuration à pas multispire, à savoir une pluralité de dépressions hélicoïdales externes et internes indépendantes (9a, 9b, 9c et 9a,i, 9b,i 9c,i), décalées l'une par rapport à l'autre dans le sens de la longueur, qui séparent les spires (4'a, 4''a, 4'''a et 4'a,i, 4''a,i, 4'''a,i) d'une égale pluralité de reliefs hélicoïdaux indépendants externes et internes, dont la surface interne délimite lesdits canaux périphériques indépendants (5'a, 5''a, 5'''a) en coopération avec la surface extérieure dudit tube central (1), cette double pluralité de dépressions hélicoïdales et de reliefs hélicoïdaux, internes et externes, étant obtenue en une seule pièce.

5. Dispositif selon l'une quelconque des revendications 3 et 4, caractérisé en ce que les portions (2, 3) terminales des extrémités distale (12) et proximale (14) du revêtement hélicoïdal périphérique (7, 7a), qui enveloppe le tube central (1), sont conformées de manière à ne pas comporter de spires et à s'appliquer par la totalité de leur surface interne, sur la surface externe des portions terminales des extrémités distale et proximale correspondantes du tube central (1), lesdites portions terminales respectives étant solidarisées entre elles par tous moyens appropriés, et notamment par collage.

6. Dispositif selon la revendication 2, caractérisé en ce que, dans le cas où le relief hélicoïdal est constitué par ladite ou lesdites tubulures hélicoïdales périphériques (10), l'extrémité distale de cette ou de ces tubulures périphériques (10) est fermée, par collage notamment, et s'applique sur l'extrémité distale correspondante dudit tube central (1) qui se prolonge dans une portion terminale ne comportant pas de spires.

7. Dispositif selon l'une quelconque des revendications 5 et 6, caractérisé en ce qu'également la portion terminale (2) du tube central (1) dépourvue de revêtement hélicoïdal, qui se trouve du côté distal (12), est pourvue d'orifices latéraux.

8. Dispositif selon l'une quelconque des revendications 1 à 6, caractérisé en ce que ledit orifice latéral (15) qui met en communication, au niveau de l'extrémité distale (12) ledit ou lesdits canaux périphériques (5a, 5b, 5'a) avec ledit canal central (6) est muni d'un clapet anti-reflux (16).

9. Dispositif selon l'une quelconque des revendications 2 à 4, caractérisé en ce que les spires (4a ou 4b ou 4'a, 4''a, 4'''a) de ladite ou desdites tubulures hélicoïdales périphériques (10) et dudit revêtement hélicoïdal (7, 7a) présentent une double paroi, de façon à délimiter deux canaux périphériques hélicoïdaux juxtaposés, à savoir un premier canal hélicoïdal compris entre la double paroi et le tube central, et un deuxième canal hélicoïdal compris dans ladite double paroi.

10. Dispositif selon la revendication 9, caractérisé en ce que le diamètre des spires est rendu variable à l'aide d'un système de réglage approprié relié, à l'aide d'un tube latéral, à ladite double paroi, à savoir audit deuxième canal hélicoïdal qui ne communique pas avec ledit premier canal hélicoïdal ni avec ledit canal central.

11. Dispositif selon la revendication 10, caractérisé en ce que le système de réglage du diamètre des spires est constitué par un système pneumatique communiquant avec ledit deuxième canal hélicoïdal, qui est compris dans ladite double paroi.

12. Dispositif selon la revendication 10, caractérisé en ce que le système de réglage du diamètre des spires est constitué par un dispositif de coulage, dans le canal hélicoïdal de la double paroi, d'une mousse en matière plastique.

13. Dispositif selon l'une quelconque des revendications 1 à 12, caractérisé en ce que la paroi du tube central (1) présente, du côté proximal (14), un épaississement (23) permettant son raccordement à un collecteur (20) du liquide biologique en dépression par l'intermédiaire d'un connecteur (19).

14. Dispositif selon l'une quelconque des revendications 1 à 13, caractérisé en ce que l'extrémité distale du tube central (1) se présente biseautée, notamment selon un profil tronconique.

**Patentansprüche**

1. Sondenartige Vorrichtung zum Zu- oder Abführen biologischer Flüssigkeiten aus einem flexiblen Material, insbesondere aus einem Silikonelastomeren, mit:
– an ihrer Aussenfläche ausgesparten und in Längsrichtung gesehen gegeneinander versetzten einer oder mehreren schraubenlinienförmigen Vertiefungen oder Rillen (9 oder 9a, 9b, 9c), die Windungen (4a oder 4b oder 4'a, 4''a, 4'''a) trennen, die zu einer oder mehreren schraubenlinienförmigen Erhöhungen mit konstanter oder veränderlicher Ganghöhe zugehörig sind, und
– ggf. mit seitlichen Öffnungen (11) zur Verbindung mit der Aussenseite der Vorrichtung, wobei die Öffnungen in der oder den schraubenlinienförmigen Rillen (9) und insbesondere am fernen Ende (12) ausgespart sind,
wobei die Vorrichtung dadurch gekennzeichnet ist, dass die oder diejenigen schraubenlinienförmigen Erhöhungen und demzufolge die Windungen (4a oder 4b oder 4'a, 4''a, 4'''a) derart hohl sind, dass die Windungen untereinander in Verbindung stehen, d.h. diejenigen, die ein und derselben Erhöhung zugehörig sind, wobei sie einen oder mehrere unabhängige umfängliche Kanäle (5a oder 5b oder 5'a, 5''a, 5'''a) umgrenzen, die jeweils am nahen Ende (14) mit dem Einlass eines seitlichen Rohres (13 oder 13a, 13b) in Verbindung stehen, das mit einem Luftanschluss und/oder einer Quelle einer Einträufel- oder Spülflüssigkeit verbunden ist, wohingegen am fernen Ende (12) zumindest einer der umfänglichen Kanäle mit der Innen- oder Aussenseite der Vorrichtung durch eine zwischenliegende seitliche Öffnung (15 oder 15b, 15c) in Verbindung steht.

2. Vorrichtung nach Anspruch 1 des Types, der ein oder mehrere aufgesetzte schraubenlinienförmige Erhöhungen aufweist, die durch ein oder mehrere unabhängige flexible Stege gebildet werden, die in Schraubenlinienform um ein zentrales Rohr (1) mit offenem fernen Ende gewickelt sind, das eine zylindrische, zylindrisch-konische, kegelstumpfartige, ovale oder andere Form aufweist und das einen mittigen Abführkanal (6) umgrenzt, und die an das zentrale Rohr (1) durch jegliche geeignete Mittel, insbesondere durch eine Klebverbindung, befestigt sind, dadurch gekennzeichnet, dass der oder die unabhängigen flexiblen Stege durch umfängliche schraubenlinienförmige Röhren (10) gebildet sind, deren Einlass den oder die unabhängigen umfänglichen Kanäle umgrenzt.

3. Vorrichtung nach Anspruch 1 des Types, der an seiner Aussenfläche eine schraubenlinienförmige Vertiefung (9) aufweist, die die Windungen (4a) einer äusseren schraubenlinienförmigen Erhöhung, die in die Wand eines umfänglichen flexiblen Rohres mit zylindrischer, zylindrisch-konischer, kegelstumpfförmiger, ovaler oder anderer Form integriert sind, trennen, dadurch gekennzeichnet, dass das umfängliche Rohr (7) an seiner Innenwandfläche mit in etwa konstanter Dicke gleichermassen eine schraubenlinienförmige Vertiefung oder Rille (9i) aufweist, die die Windungen (4a, i) einer inneren schraubenlinienförmigen Erhöhung trennt, wobei die innere schraubenlinienförmige Vertiefung bzw. Erhöhung der äusseren schraubenlinienförmigen Erhöhung bzw. Vertiefung (9) entspricht, und dass das umfängliche Rohr (7), das auf der Innenfläche gleichermassen eine solche schraubenlinienförmige Konfiguration aufweist, einen schraubenlinienförmigen Überzug mit einfachem Schraubengang für ein zentrales Rohr (1) mit fernem, offenem Ende darstellt, das einen mittigen Kanal (6) umgrenzt und sich an die Form des umfänglichen Rohres (7) anpasst und dessen Aussendurchmesser in etwa gleich dem Innendurchmesser des umfänglichen Rohres ist, den dieses in Höhe der inneren Erhöhungen aufweist, und zwar derart, dass der umfängliche Kanal (5a) zwischen der Aussenfläche des zentralen Rohres (1) und der Fläche, die die innere schraubenlinienförmige Vertiefung (9i) umgrenzt, aufgenommen ist, wobei die Verbindung zwischen dem zentralen Rohr (1) und dem umfänglichen Rohr (7) durch jedes geeignete Mittel, insbesondere durch Verklebung, entsprechend der äusseren schraubenlinienförmigen Vertiefung (9), bzw. entsprechend der inneren schraubenlinienförmigen Erhöhung erreichbar ist.

4. Vorrichtung nach Anspruch 1, dadurch gekennzeichnet, dass sie einen schraubenlinienförmigen Überzug enthält, der durch ein umfängliches flexibles Rohr (7a) gebildet ist, das ein zentrales Rohr (1) der zuvor genannten Art umschliesst und an seiner Aussen- und an seiner Innenfläche eine Konfiguration mit einem vielwindigen Verlauf aufweist, d.h. eine Vielzahl an unabhängigen äusseren und inneren schraubenlinienförmigen Vertiefungen (9a, 9b, 9c und 9a, i, 9, b, i, 9c, i), die in Längsrichtung gesehen gegeneinander versetzt sind, die die Windungen (4'a, 4''a, 4'''a und 4'a, i, 4''a, i, 4'''a, i) durch eine gleiche Vielzahl an unabhängigen schraubenlinienförmigen äusseren und inneren Erhöhungen trennen, deren Innenfläche die unabhängigen umfänglichen Kanäle (5'a, 5''a, 5'''a) in Zusammenhang mit der Aussenfläche des zentralen Rohres (1) umgrenzen, wobei die doppelte Vielzahl an schraubenlinienförmigen inneren und äusseren Vertiefungen und Erhöhungen durch ein einziges Stück erhalten ist.

5. Vorrichtung nach einem der Ansprüche 3 und 4, dadurch gekennzeichnet, dass die Endbereiche (2, 3) der fernen (12) und nahen (14) Enden des umfänglichen schraubenlinienförmigen Überzuges (7, 7a), der das zentrale Rohr (1) umschliesst, derart ausgebildet sind, dass sie keine Windungen tragen und mit ihrer gesamten Innenfläche auf die Aussenflächen der entsprechenden Endbereiche der nahen und fernen Enden des zentralen Rohres (1) passen, wobei die entsprechenden Endbereiche durch geeignete Mittel und insbesondere durch Verkleben miteinander verbunden sind.

6. Vorrichtung nach Anspruch 2, dadurch gekennzeichnet, dass, falls die schraubenlinienförmige Erhöhung durch die oder diejenigen umfänglichen schraubenlinienförmigen Röhren (10) gebildet ist, das ferne Ende des oder der umfänglichen Rohre (10), insbesondere durch Verkleben geschlossen ist und auf das entsprechende ferne Ende des zentralen Rohres (1), das sich in einem Endbereich, der keine Windungen trägt, verlängert, passt.

7. Vorrichtung nach einem der Ansprüche 5 und 6, dadurch gekennzeichnet, dass der sich auf der fernen Seite (12) befindliche Endbereich (2) des zentralen Rohres (1), der nicht mit dem schraubenlinienförmigen Überzug versehen ist, mit seitlichen Öffnungen versehen ist.

8. Vorrichtung nach einem der Ansprüche 1 bis 6, dadurch gekennzeichnet, dass die seitliche Öffnung (15), die auf Höhe des fernen Endes (12) den oder die umfänglichen Kanäle (5a, 5b, 5'a) mit dem mittigen Kanal (6) in Verbindung setzen, mit einem Rückflusssperrventil (16) versehen ist.

9. Vorrichtung nach einem der Ansprüche 2 bis 4, dadurch gekennzeichnet, dass die Windungen (4a oder 4b oder 4'a, 4''a, 4'''a) des oder der umfänglichen schraubenlinienförmigen Rohre (10) und des schraubenlinienförmigen Überzuges (7, 7a) eine derartige Doppelwand darstellen, dass zwei nebeneinander liegende schraubenlinienförmige umfängliche Kanäle gebildet sind, d.h. ein erster schraubenlinienförmiger Kanal, der zwischen der Doppelwand und dem zentralen Rohr gelegen ist, und ein zweiter schraubenlinienförmiger Kanal, der in der Doppelwand enthalten ist.

10. Vorrichtung nach Anspruch 9, dadurch gekennzeichnet, dass der Durchmesser der Windungen mit Hilfe eines geeigneten Regelungssystemes variierbar gemacht ist, das mit Hilfe eines seitlichen Rohres mit der Doppelwand verbunden ist, d.h. an den zweiten schraubenlinienförmigen Kanal, der weder mit dem ersten schraubenlinienförmigen Kanal noch mit dem mittigen Kanal verbunden ist.

11. Vorrichtung nach Anspruch 10, dadurch gekennzeichnet, dass das Regelungssystem für den Durchmesser der Windungen durch ein pneumatisches System gebildet ist, das mit dem zweiten schraubenlinienförmigen Kanal in Verbindung steht, der in der Doppelwand aufgenommen ist.

12. Vorrichtung nach Anspruch 10, dadurch gekennzeichnet, dass das Regelungssystem für den Durchmesser der Windungen durch eine Vorrichtung gebildet ist, die in den schraubenlinienförmigen Kanal der Doppelwand einen Schaum aus Kunststoffmaterial spritzt.

13. Vorrichtung nach einem der Ansprüche 1 bis 12, dadurch gekennzeichnet, dass die Wand des zentralen Rohres (1) an der nahen Seite (14) eine Verdickung (23) aufweist, die dessen Befestigung durch Zwischenlegen eines Verbindungsstückes (19) mit einem unter Unterdruck stehenden Sammelgefäss (20) für eine biologische Flüssigkeit ermöglicht.

14. Vorrichtung nach einem der Ansprüche 1 bis 13, dadurch gekennzeichnet, dass das ferne Ende des zentralen Rohres (1) insbesondere mit einem kegelstumpfförmigen Profil abgeschrägt ist.

**Claims**

1. Probe device for the channeling or draining of a biological fluid of the type made of a flexible material, notably silicon elastomer, and having:
— one or several helical depressions or grooves (9 or 9a, 9b, 9c) with being provided in its outer surface and staggered with respect to one another in the lengthwise direction, separate turns (4a or 4b or 4'a, 4''a, 4'''a) belonging to one or several helical bulges of constant or variable pitch, and
— possibly side openings (11) communicating with the outside of the device, said openings being arranged in said helical depressions or depression (9), and in particular at the distal end,
wherein said helical bulges, and therefore said turns (4a or 4b or 4'a, 4''a, 4'''a) are hollow, so that the turns intercommunicating with one another, that is to say belonging to a same bulge, define or one or several independant peripheral ducts (5a or 5b or 5'a, 5''a, 5'''a), each communicating, at the proximal extremity (14), with the opening of a side nozzle (13 or 13a, 13b) connected to a ventilation aperture and/or to irrigation or instillation liquid source, whereas at the distal end (12) at least one of said peripheral ducts communicates with the inside or the outside of the device by means of a side opening (15 or 15b, 15c).

2. Device in accordance with claim 1, of the type having one or several added helical bulges, that is to say formed by one or several flexible independent threads coiled in a helix about the central tube (1) open at the distal end, of cylindrical, cylindro-conical, tapered, oval or other form, and delimiting a central drainage channel (6), and which are fixed to this central tube (1) by any appropriate means and in particular by glueing, wherein said independent flexible thread or threads are formed by peripheral helical tubes (10) the opening of which defines said independent peripheral duct or ducts.

3. Device in accordance with claim 1, of the type having on its outer surface a helical depression (9) separating the turns (4a) of an outer helical bulge integral with the wall of a flexible peripheral tube, of cylindrical, cylindrico-conical, tapered, oval or other form wherein this peripheral tube (7) also has on the inner surface of its approximately uniform thickness wall, a helical depression or groove (9i) separating the turns (4a,i) of an inner helicoidal bulge, said depression and this helical bulge corresponding, respectively, to said outer helical bulge and depression (9), and wherein the peripheral tube (7) also having such a helical configuration on its inner surface forms a simple helical covering for a central tube (1) with an open distal end which limits a central channel (6) and adapts itself to the form of the peripheral tube (7), the outer diameter of which being roughly equal to the inner diameter presented by the peripheral tube at the level of said inner bulge, so that said peripheral channel (5a) is comprised between the outer surface of the central tube (11) and the surface delimiting said inner helical depression (9i), the fixing of the central tube (11) to the peripheral tube (7) being obtained by any appropriate means, notably by glueing, in correspondance with said outer helical depression (9), that is to say in correspondance with said inner helical bulge.

4. Device in accordance with claim 1, characterized in that it comprises a helical covering made up of a peripheral flexible tube (7a) enveloping a central tube (1) of the above mentioned type and presenting on its outer surface and on its inner surface a multihelical pitch configuration, that is to say a plurality of independent inner and outer helical depressions (9a, 9b, 9c and 9a,i, 9b,i, 9c,i) staggered with respect to one another in the direction of the length, which separates the turns (4'a, 4''a, 4'''a and 4'a,i, 4''a,i, 4'''a,i) by an equal plurality of inner and outer independent helical bulges, the inner surface of which delimites said independent peripheral channels (5'a, 5''a, 5'''a) in cooperation with the outer surface of said central tube (1), said double plurality of inner and outer helical depressions and bulges being formed from one single piece.

5. Device in accordance with any one of claims 3 and 4, wherein the end parts (2, 3) of the distal (12) and proximal (14) extremities of the peripheral helicoidal covering (7, 7a) which envelops the central tube (1) are formed in such a way as not to comprise turns and to apply themselves by the totality of their inner surface on the external surface of the terminal parts of the corresponding proximal and distal extremities of the central tube (1), said respective terminal portions being fixed to one another by any appropriate means, and in particular by glueing.

6. Device in accordance with claim 2, wherein, in the case where the helicoidal bulge is formed by said peripheral helical tube, or tubes (10), the distal extremity of this peripheral tube or tubes (10) is closed, by glueing notably, and applies itself on the corresponding distal extremity of said central tube (1) which extends in a terminal part with no turns.

7. Device in accordance with any one of the claims 5 and 6, wherein the terminal part (2) of the central tube (1) having no helical covering positioned at the distal side (12), is provided with lateral openings.

8. Device in accordance with any of claims 1 to 6, wherein said lateral opening (15) communicating at the level of the distal end (12) of said peripheral channel or channels with said central channel (6) is provided with an anti flow-back flap (16).

9. Device in accordance with any one of claims 2 to 4, wherein the turns (4a or 4b or 4'a, 4''a, 4'''a) of said peripheral helical tube or tubes (10) and of said helical covering (7, 7a) have a double wall, so as to delimit two juxtaposed helical peripheral channels, that is to say a first helical channel comprised between the double wall and the central tube and a second helical channel comprised in said double wall.

10. Device in accordance with claim 9, wherein the diameter of the turns is rendered variable by means of an appropriate regulating system connected with the aid of a lateral tube, to the double wall, that is to say, to said second helical channel not communicating with said first helical channel nor with said central channel.

11. Device in accordance with claim 10, wherein the turn diameter regulating system is made up of a pneumatic system communicating with said second helical channel comprised in said double wall.

12. Device in accordance with claim 10, wherein the turn diameter regulating system is formed by a device for flowing a foam of plastics material into the helical channel of the double wall.

13. Device in accordance with any one of claims 1 to 12, wherein the wall of the central tube (1) presents, on the proximal side (14), a thickening (23) enabling its connection with a biological fluid collector (20) under vacuum by the intermediary of a connector (19).

14. Device in accordance with any one of claims 1 to 13, wherein the distal extremity of the central tube (1) is bevelled, notably in accordance with a tapered profile.

FIG. 1

# FIG. 2

# FIG.3

FIG.4